(19) Europäisches Patentamt
European Patent Office
Office européen des brevets

(11) Veröffentlichungsnummer : **0 451 720 B1**

(12) # EUROPÄISCHE PATENTSCHRIFT

(45) Veröffentlichungstag der Patentschrift :
**16.02.94 Patentblatt 94/07**

(51) Int. Cl.[5] : **C07C 25/08,** C07C 17/38

(21) Anmeldenummer : **91105399.9**

(22) Anmeldetag : **05.04.91**

(54) **Verfahren zur Trennung von m- und p-Dichlorbenzol.**

Die Akte enthält technische Angaben, die nach dem Eingang der Anmeldung eingereicht wurden und die nicht in dieser Patentschrift enthalten sind.

(30) Priorität : **10.04.90 DE 4011501**

(43) Veröffentlichungstag der Anmeldung :
**16.10.91 Patentblatt 91/42**

(45) Bekanntmachung des Hinweises auf die Patenterteilung :
**16.02.94 Patentblatt 94/07**

(84) Benannte Vertragsstaaten :
**BE CH DE ES FR GB IT LI NL**

(56) Entgegenhaltungen :
**EP-A- 0 012 891**
**DE-A- 2 332 889**

(56) Entgegenhaltungen :
**CHEMICAL ABSTRACTS, Band 100, Nr. 7, 13. Februar 1984, Columbus, Ohio, USA; "Separation of dichlorobenzene isomers", Seite 549, Zusammenfassung Nr. 51 226u; & JP-A-58 174 336**
**PATENT ABSTRACTS OF JAPAN, unexamined applications, C Sektion, Band 4, Nr. 22, 23. Februar 1980, THE PATENT OFFICE JAPANESE GOVERNMENT, Seite 47 C 74**
**PATENT ABSTRACTS OF JAPAN, unexamined applications, C Feld, Band 8, Nr. 7, 12. Januar 1984, THE PATENT OFFICE JAPANESE GOVERNMENT ; Seite 35 C 204**

(73) Patentinhaber : **HOECHST AKTIENGESELLSCHAFT**
**D-65926 Frankfurt (DE)**

(72) Erfinder : **Rittner, Siegbert, Dr.**
**Kornblumenweg 5**
**W-6082 Mörfelden-Walldorf (DE)**
Erfinder : **Schmidt, Adolf, Dr.**
**Hainerweg 23**
**D-6238 Hofheim am Taunus (DE)**
Erfinder : **Steiner, Rudolf, Prof. Dr.**
**Rehweiher-Strasse 14**
**W-8520 Erlangen-Kosbach (DE)**
Erfinder : **Unverdorben, Leonhard**
**Schleifweg 4**
**W-8525 Uttenreuth (DE)**

EP 0 451 720 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein Verfahren zur Trennung von m- und p-Dichlorbenzol durch extraktive Rektifikation mit einem Extraktionsmittel.

Die Gewinnung von m-Dichlorbenzol (m-DCB) aus Gemischen mit o- und p-Dichlorbenzol (p-DCB) gehört zu den schwierigsten Trennaufgaben der aromatischen Zwischenproduktchemie. Denn die Siedepunkte zwischen dem m- und dem p-DCB liegen nur um weniger als 0,2°C auseinander, so daß die Abtrennung eines reinen, über 99 %igen m-DCB durch fraktionierte Rektifikation praktisch unmöglich ist.

Wegen dieser Trennschwierigkeit werden, um zu einem wenigstens nahezu 98 %igen m-DCB zu gelangen, in der Technik gelegentlich auch komplizierte Umwege in Kauf genommen. So wird beispielsweise in Winnacker/Küchler, Chemische Technologie, 4. Aufl., Bd. 6, S. 158 (1982) ein Weg beschrieben, bei dem über m-Dinitrobenzol oder m-Chlorbenzolsulfochlorid gezielt m-Dichlorbenzol hergestellt wird. In der US-PS 3 170 961 wird der Umweg über Brom-Isomere empfohlen, das darin besteht, daß man die Dichlorbenzole bromiert, die Brom-Isomeren durch Destillation trennt und dann das Brom so abspaltet, daß man die ursprünglichen Dichlorbenzole zurückerhält. Dieses Verfahren ist jedoch schwierig und teuer. Auch eine Schmelzkristallisation, wie sie für die Trennung von vielen aromatischen Isomeren mit Erfolg angewendet wird, ist bei m-/p-DCB-Gemischen unwirtschaftlich, da die Kristallisation von reinem m-DCB tiefe Temperaturen (ca. -30°C) und sehr hohe Konzentrationen von über 88 % m-DCB im Einsatzgemisch erfordert und ein hochprozentiges eutektisches Gemisch mit 88 % m-DCB liefert.

Da m-DCB ein wirtschaftlich bedeutendes Zwischenprodukt darstellt, das als Synthesebaustein für eine Reihe wichtiger Folgeprodukte dient, sind immer wieder neue Trennvorschläge gemacht worden. So ist z.B. ein Verfahren mit Hilfe von Molekularsieben in der US-PS 2 958 708 beschrieben. Dieses an sich interessante Verfahren, das zu den gewünschten Reinheiten von über 99 % m-DCB führt, hat jedoch den Nachteil der großen Schwierigkeiten, die beim Regenerieren des Molekularsiebs auftreten, und der hohen Kosten.

Eine besonders attraktive Trenntechnik wird in der Praxis in der Extraktiv-Rektifikation gesehen, bei der mit Hilfe von Extraktionsmitteln als spezifischen Hilfsstoffen eine Hochreinigung von m-DCB ermöglicht wird.

So wird beispielsweise in der DE-PS 23 32 889 Hexamethylphosphorsäuretriamid, eine kanzerogen wirkende Substanz, als ein solcher Hilfsstoff beschrieben. Als weitere wirksame Extraktionsmittel werden dort aufgrund guter Werte für den Trennfaktor (die relative Flüchtigkeit) im Dreistoffsystem die Extraktionsmittel Dimethylsulfoxid, N-Methylpyrrolidon und Dibutysulfoxid genannt. Des weiteren werden in den japanischen Patentanmeldungen JP-A 54 160 322 und JP-A 58 174 333 u.a. solche Hilfsstoffe wie o-, m- und p-Kresol und Anilin-Derivate empfohlen. Diese Stoffe sind jedoch mehr oder weniger toxisch, wirken korrodierend oder ihre Siedepunkte liegen ungünstig. Darüber hinaus sind einige dieser Hilfsstoffe thermisch wenig stabil oder ihre thermische Langzeitstabilität ist nicht ausreichend, so daß sie nicht mehrfach eingesetzt werden können, was aber für praktische Zwecke wichtig ist.

Es bestand daher die Aufgabe, ein Verfahren zur Trennung von m- und p-Dichlorbenzol zur Verfügung zu stellen, das die Nachteile des Standes der Technik nicht aufweist und das insbesondere mit toxikologisch und ökologisch unbedenklichen, thermisch stabilen und nicht korrodierend wirkenden Extraktionsmitteln arbeitet und welches das m-Isomere mit einer Reinheit von über 99 % zu gewinnen gestattet. Weiterhin sollte dieses Extraktionsmittel höher sieden als das zu trennende Gemisch und eine leicht dosierbare, nicht viskose und preiswerte Flüssigkeit sein.

Es wurde nun gefunden, daß Alkylencarbonate, wie Äthylencarbonat und Propylencarbonat, für diesen Zweck hervorragend geeignet sind.

Die Erfindung betrifft daher ein Verfahren zur Trennung von m- und p-Dichlorbenzol durch extraktive Rektifikation mit einem Extraktionsmittel und Abtrennung dieses Extraktionsmittels, wobei man als Extraktionsmittel Alkylencarbonate der Formel I (siehe Patentanspruch 1) verwendet, worin $R^1$, $R^2$, $R^3$ und $R^4$ untereinander gleich oder verschieden sind und für Wasserstoff, einen Methyl- oder einen Äthylrest stehen, mit der Maßgabe, daß die Reste $R^1$ bis $R^4$ zusammen nicht mehr als 6 C-Atome enthalten.

Erfindungsgemäß ist es möglich, nicht nur aus m-/p-DCB-Gemischen mit hohem m-DCB-Gehalt m-DCB-Destillate mit Reinheiten von nahezu 100 % abzutrennen, sondern überraschenderweise auch beispielsweise aus solchen Gemischen, die nur 50 % m-DCB enthalten, wie sie z.B. bei der katalytischen Isomerisierung anderer Dichlorbenzole anfallen. Verständlicherweise benötigt man zur Trennung eines nur 50 % m-DCB enthaltenden Gemisches eine Kolonne mit entsprechend höherer Trennstufenzahl als bei der Einspeisung eines Gemisches mit 80 % m-DCB. Das Isomerengemisch kann neben diesen beiden DCB-Isomeren noch andere chlorierte Benzole und/oder chlorierte Nitrobenzole in kleinen Mengen enthalten. Diese Verbindungen sind im allgemeinen Monochlorbenzol, ortho-Dichlorbenzol und Trichlorbenzol, die bei der Herstellung von Dichlorbenzol anfallen.

Als Extraktionsmittel werden Alkylencarbonate der Formel (I), wie beispielsweise Äthylen-, Propylen- und

Butylencarbonat verwendet. Besonders bevorzugt sind Äthylen- und Propylencarbonat, deren Siedepunkte in genügend weitem aber nicht zu großem Abstand von denen der m-/p-Dichlorbenzole liegen. Es können auch entsprechende Gemische zum Einsatz kommen.

Zum Beweis der Wirksamkeit der erfindungsgemäß verwendeten Extraktionsmittel sind in nachfolgender Tabelle Meßwerte für den Nutzeffekt β, das ist der Faktor, um den der Trennfaktor (die relative Flüchtigkeit) von m- gegenüber p-Dichlorbenzol erhöht wird, zusammengestellt. Diese Meßwerte wurden bei einer Isomerenkonzentration von je 10 Vol.-% im Extraktionsmittel und einer Temperatur von 120°C erhalten. Zum Vergleich mit der Wirksamkeit dieser Extraktionsmittel sind auch die entsprechenden Meßwerte für Hexamethylphosphortriamid und Sulfolan in die Tabelle aufgenommen.

Tabelle

| Extraktionsmittel | Nutzeffekt β |
|---|---|
| Hexamethylphosphortriamid | 1,22 |
| Sulfolan | 1,16 |
| Äthylencarbonat | 1,17 |
| Propylencarbonat | 1,19 |

Der Nutzeffekt β wurde mit der gaschromatographischen Dampfraummethode gemessen, die bereits früher in Verfahrenstechnik 8 (1974) Nr. 12, S. 343-347 beschrieben ist.

Die Prüfungen ergaben, daß Äthylencarbonat und Propylencarbonat ähnlich gute Nutzeffekte wie das toxische bzw. thermisch instabile Hexamethylphosphortriamid und das zu hoch siedende, geruchsintensive Sulfolan aufweisen.

Diese Carbonate sind daher und aufgrund ihrer sonstigen, überwiegend erst im Rahmen dieser Erfindung ermittelten positiven Stoffeigenschaften (auch bei längerem Einsatz nicht korrodierend sowie chemisch und thermisch stabil, leicht abtrennbar von Dichlorbenzolen u.a.) und nicht zuletzt auch wegen ihrer Nichttoxizität als Extraktionsmittel für eine Extraktivrektifikation von m-/p-Dichlorbenzolgemischen ausgezeichnet geeignet.

Zweckmäßig wird das erfindungsgemäße Verfahren so durchgeführt, daß die Extraktionskolonne bei niedrigem Druck, vorzugsweise etwa 50 bis etwa 300 hPa, insbesondere etwa 80 bis etwa 150 hPa, gemessen am Kolonnenkopf, mit möglichst kleinem Druckverlust zwischen Kolonnenfuß und -kopf, vorzugsweise etwa 0 bis etwa 100 hPa, insbesondere etwa 10 bis etwa 70 hPa, und mit möglichst großer Trennstufenzahl, vorzugsweise mindestens etwa 20 bis etwa 250, insbesondere mindestens etwa 80 bis etwa 180 theoretischen Trennstufen, betrieben wird. Das Rücklaufverhältnis (Rücklauf:Destillat) beträgt zweckmäßig etwa 2:1 bis etwa 30:1, insbesondere etwa 4:1 bis etwa 25:1, und das Zulaufverhältnis von Zulaufmenge des Extraktionsmittels zu Zulaufmenge des m-/p-DCB-Gemischs beträgt bevorzugt etwa 4:1 bis etwa 40:1, insbesondere etwa 9:1 bis etwa 30:1. Im allgemeinen weist das in die Extraktionskolonne eingespeiste, zu trennende Gemisch eine Temperatur auf, die in Abhängigkeit vom gewählten Druck im allgemeinen zwischen 60 bis 150°C, vorzugsweise 70 bis 120°C und insbesondere 75 bis 115°C liegt. Die Temperatur der Extraktionsmittel liegt gleichfalls in diesen Bereichen und ist in der Regel gleich derjenigen des zu trennenden Gemischs. Die Sumpftemperatur in der Extraktionskolonne sollte zweckmäßigerweise 180°C, vorzugsweise 160°C nicht übersteigen.

In einer bevorzugten Ausführungsform wird die erfindungsgemäße Extraktiv-Rektifikation mit einer Schmelzkristallisation kombiniert. Ist das m-Isomere als besonders reines Produkt gewünscht, kann es nämlich wirtschaftlich sein, durch extraktive Rektifikation ein m-DCB-Destillat zu erzeugen, das noch durch 2 bis 7 % p-DCB verunreinigt ist und durch eine nachgeschaltete, einstufige Schmelzkristallisation das Destillat in 100 %iges m-DCB-Kristallisat und eutektische Schmelze (88 % m-, 12 % p-DCB) zu trennen und anschließend diese Schmelze in die Extraktiv-Rektifikation zurückzuführen. Beispielsweise können aus 1000 kg Destillat, das 950 kg m-DCB und 50 kg kristallines p-DCB enthält, 580 kg 100 %iges m-DCB abgetrennt werden. Die Restschmelze von 420 kg (88 % m-DCB) wird in die Extraktiv-Rektifikation zurückgegeben.

**Beispiele**

1. Eine Laborkolonne mit 50 mm Innendurchmesser, mit innen verspiegeltem, außen beheiztem Vakuummantel, die mit SULZER-Laborpackung EX gepackt ist und eine trennwirksame Höhe von 3,50 m aufweist, wird in 1,00 m Höhe mit dem zu trennenden Gemisch,bestehend aus 87,5 % m-, 2,5 % p- und 10,0 % o-Dichlorbenzol,mit einer Temperatur von 75°C und in 3,50 m Höhe mit dem Extraktionsmittel Propylencarbonat, gleichfalls mit einer Temperatur von 75°C,und dem Rücklauf gespeist. Der Druck am Kolonnenkopf beträgt 100,0 hPa. Das Extraktionsmittel verläßt den Verdampfer am Kolonnenfuß flüssig siedend beinahe quantitativ (99,8 % der Einspeisung) zusammen mit wenig m-Dichlorbenzol und praktisch dem gesamten Anteil von Verunreinigung aus p- und o-Dichlorbenzol, während am Kopf der Kolonne dampfförmig m-Dichlorbenzol abgezogen wird, das nur durch 0,2 % p-Dichlorbenzol und 3 % leicht abtrennbares Extraktionsmittel Propylencarbonat verunreinigt ist. Der Rücklauf bestehend aus Dampfkondensat, das am Kolonnenkopf anfällt, wird auf 7,2 g/min eingestellt, was einem Rücklaufverhältnis von Rücklauf:Destillat = 9:2 entspricht, während 2,00 g/min zu trennendes Gemisch und 28,00 g/min Propylencarbonat eingespeist und 1,60 g/min Destillat und 28,40 g/min Sumpf erzeugt werden.

2. In dieselbe Kolonne, wie in Beispiel 1 beschrieben, wird ein anderes Gemisch, bestehend aus 50,4 % m- und 49,6 % p-Dichlorbenzol in 1 m Höhe eingespeist und mit Hilfe von Propylencarbonat, das zusammen mit dem Rücklauf in 3,5 m Höhe aufgegeben wird, getrennt. Der Druck am Kolonnenkopf liegt wiederum bei 100,0 hPa, die Temperatur von zu trennendem Gemisch und Extraktionsmittel beträgt 105°C. Bei einem Rücklaufverhältnis von Rücklauf:Destillat = 9:1 werden 2,00 g/min zu trennendes Gemisch und 28,00 g/min Propylencarbonat eingespeist und 0,95 g/min Destillat und 29,05 g/min Sumpf erzeugt. Das Destillat enthält 91,3 % m- und 5,7 % p-Dichlorbenzol sowie 3,0 % Propylencarbonat.

Die Abtrennung des Extraktionsmittels aus dem Sumpfprodukt stellt keine schwierige Trennaufgabe dar und wird gewöhnlich in einer nachgeschalteten Kolonne durchgeführt, so daß das Extraktionsmittel befreit von Leichtsiedern, in die Extraktionskolonne zurückgespeist werden kann. Aufgrund der Beispiele läßt sich berechnen, in welchem Maße die wirksame Trennstufenhöhe der Kolonne vergrößert werden muß, um ein über 99 %iges m-Produkt zu bekommen.

3. Dieselbe Kolonne, wie in Beispiel 1 beschrieben, wird für einen weiteren Versuch um zwei weitere Kolonnenschüsse vom gleichen Typ wie die schon vorhandenen ergänzt auf insgesamt 5,00 m trennwirksame Höhe. Sie wird gespeist in 1,00 m Höhe mit zu trennendem Gemisch, bestehend aus 70,0 % m-, 29,8 % p- und 0,2 % o-Dichlorbenzol, in 4,00 m Höhe mit dem Extraktionsmittel Propylencarbonat und in 5,00 m Höhe mit dem Rücklauf. Das zu trennende Gemisch wird mit einer Temperatur von 95°C und einer Menge von 2,00 g/min eingespeist, während das Extraktionsmittel mit 95°C und 45,00 g/min gefördert wird. Der Rücklauf wird mit 75°C und 20,0 g/min aufgegeben. Das Rücklaufverhältnis liegt bei 9:1. Der Kopfdruck beträgt 100,0 hPa. Die Rektifikation liefert bei einer Kopfdampftemperatur von 98°C 1,12 g/min Destillat mit einem Gehalt von 99,2 % m-Dichlorbenzol. Der Sumpf fällt mit 155°C in einer Menge von 45,88 g/min an und besteht zu 98 % aus Propylencarbonat und zu 2 % aus Dichlorbenzolen.

4. In dieselbe Kolonne, wie in Beispiel 1 beschrieben, wird in 1,00 m Höhe eine Menge von 2,0 g/min eines zu trennenden Gemisches aus 72,9 % m- und 27,1 % p-Dichlorbenzol eingespeist, während in 3,00 m Höhe 20 g/min Ethylencarbonat und am Kolonnenkopf der aus dem Dampfkondensat bestehende Rücklauf aufgegeben werden. Der Druck am Kolonnenkopf beträgt 100,0 hPa. Bei einem Rücklaufverhältnis von Rücklauf:Destillat = 11:1 wird 1,3 g/min Destillat erzeugt, das 94,5 % m-, 4,2 % p-Dichlorbenzol und 1,3 % Ethylencarbonat enthält. Der aus dem Verdampfer abgezogene Sumpfstrom von 20,7 g/min besteht überwiegend aus dem Hilfsstoff Ethylencarbonat, der durch eine einfache thermische Regeneration von den Resten an m- und p-Dichlorbenzol getrennt werden kann.

## Patentansprüche

1. Verfahren zur Trennung von m- und p-Dichlorbenzol durch extraktive Rektifikation mit einem Extraktionsmittel und Abtrennung dieses Extraktionsmittels, dadurch gekennzeichnet, daß als Extraktionsmittel Alkylencarbonate der allgemeinen Formel

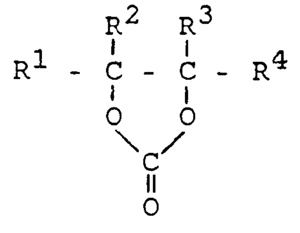

(I)

verwendet werden, worin $R^1$, $R^2$, $R^3$ und $R^4$ untereinander gleich oder verschieden sind und für Wasserstoff, einen Methyl- oder einen Äthylrest stehen, mit der Maßgabe, daß die Reste $R^1$ bis $R^4$ zusammen nicht mehr als 6 C-Atome enthalten.

2. Verfahren nach Anspruch 1, dadurch gekennzeichnet, daß das Alkylencarbonat Äthylen- und/oder Propylencarbonat ist.

3. Verfahren nach Anspruch 1 oder 2, dadurch gekennzeichnet, daß die Extraktionskolonne bei niedrigem Druck mit möglichst kleinem Druckverlust zwischen Kolonnenfuß und -kopf und mit möglichst großer Trennstufenzahl betrieben wird.

4. Verfahren nach Anspruch 3, dadurch gekennzeichnet, daß die Extraktionskolonne bei einem Druck am Kolonnenkopf von etwa 50 bis etwa 300 hPa, mit einem Druckverlust zwischen Kolonnenfuß und -kopf von 0 bis etwa 100 hPa und mit einer Trennstufenzahl von mindestens 20 theoretischen Trennstufen betrieben wird.

5. Verfahren nach Anspruch 3, dadurch gekennzeichnet, daß die Extraktionskolonne bei einem Druck am Kolonnenkopf von 80 bis 150 hPa, mit einem Druckverlust zwischen Kolonnenfuß und -kopf von 10 bis 70 hPa und mit einer Trennstufenzahl von mindestens 80 theoretischen Trennstufen betrieben wird.

6. Verfahren nach einem oder mehreren der Ansprüche 1 bis 5, dadurch gekennzeichnet, daß das Rücklaufverhältnis (Rücklauf:Destillat) 2:1 bis 30:1 ist.

7. Verfahren nach einem oder mehreren der Ansprüche 1 bis 5, dadurch gekennzeichnet, daß das Rücklaufverhältnis (Rücklauf:Destillat) 4:1 bis 25:1 ist.

8. Verfahren nach einem oder mehreren der Ansprüche 1 bis 7, dadurch gekennzeichnet, daß das Zulaufverhältnis von Zulaufmenge des Extraktionsmittels zu Zulaufmenge des m-/p-Dichlorbenzolgemisches 4:1 bis 40:1 beträgt.

9. Verfahren nach einem oder mehreren der Ansprüche 1 bis 7, dadurch gekennzeichnet, daß das Zulaufverhältnis von Zulaufmenge des Extraktionsmittels zu Zulaufmenge des m-/p-Dichlorbenzolgemisches 9:1 bis 30:1 beträgt.

10. Verfahren nach einem oder mehreren der Ansprüche 1 bis 9, dadurch gekennzeichnet, daß die Extraktiv-Rektifikation mit einer Schmelzkristallisation kombiniert wird.

## Claims

1. A process for separating m- and p-dichlorobenzene by extractive rectification with an extractant and removal of this extractant, which comprises using an extractant that is an alkylene carbonate of the formula

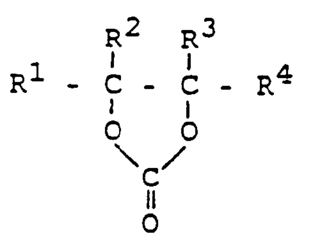

(I)

where $R^1$, $R^2$, $R^3$ and $R^4$ are identical to or different from one another and each is hydrogen, methyl or ethyl, with the proviso that together the radicals $R^1$ to $R^4$ do not contain more than 6 carbon atoms.

2. The process of claim 1, wherein the alkylene carbonate is ethylene carbonate and/or propylene carbonate.

3. The process of claim 1 or 2, wherein the extraction column is operated at a low pressure with a very small pressure loss between column base and top and with a very large number of separating stages.

4. The process of claim 3, wherein the extraction column is operated at a top of column pressure of about

50 to about 300 hPa, with a pressure loss between column base and top of 0 to about 100 hPa and with at least 20 theoretical separating stages.

**5.** The process of claim 3, wherein the extraction column is operated at a top of column pressure of 80 to 150 hPa, with a pressure loss between column base and top of 10 to 70 hPa and with at least 80 theoretical separating stages.

**6.** The process of one or more of claims 1 to 5, wherein the reflux ratio (reflux:distillate) is 2:1 to 30:1.

**7.** The process of one or more of claims 1 to 5, wherein the reflux ratio (reflux:distillate) is 4:1 to 25:1.

**8.** The process of one or more of claims 1 to 7, wherein the ratio of extractant feed rate to the feed rate for the m-/p-dichlorobenzene mixture is 4:1 to 40:1.

**9.** The process of one or more of claims 1 to 7, wherein the ratio of extractant feed rate to the feed rate for the m-/p-dichlorobenzene mixture is 9:1 to 30:1.

**10.** The process of one or more of claims 1 to 9, wherein the extractive rectification is combined with a melt crystallization.

## Revendications

**1.** Procédé de préparation du m-dichlorobenzène d'avec le p-dichlorobenzène par une rectification extractive avec un agent d'extraction puis séparation de cet agent, procédé caractérisé en ce que l'on utilise comme agent d'extraction un carbonate d'alkylène de formule I ci-dessous :

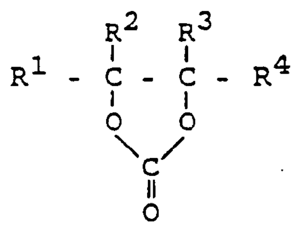

(I)

formule dans laquelle $R^1$, $R^2$, $R^3$ et $R^4$, qui peuvent être identiques ou différents les uns des autres, représentent l'hydrogène ou le groupe méthyle ou éthyle, avec la condition qu'ils n'aient pas au total plus de 6 atomes de carbone.

**2.** Procédé selon la revendication 1, caractérisé en ce que le carbonate d'alkylène est du carbonate d'éthylène et/ou de propylène.

**3.** Procédé selon la revendication 1 ou 2, caractérisé en ce que l'on fait fonctionner la colonne d'extraction à une pression basse, avec la plus faible perte de pression possible entre le pied et la tête de colonne et avec le plus grand nombre possible d'étages de séparation.

**4.** Procédé selon la revendication 3, caractérisé en ce que l'on fait fonctionner la colonne d'extraction à une pression de tête d'environ 50 à 300 hPa, avec une perte de pression entre le pied et la tête de colonne de 0 à environ 100 hPa et avec un nombre d'étages de séparation d'au moins 20 étages théoriques.

**5.** Procédé selon la revendication 3, caractérisé en ce que l'on fait fonctionner la colonne d'extraction à une pression de tête de 80 à 150 hPa, avec une perte de pression entre le pied et la tête de colonne de 10 à 70 hPa et avec un nombre d'étages de séparation d'au moins 80 étages théoriques.

**6.** Procédé selon une ou plusieurs des revendications 1 à 5, caractérisé en ce que le rapport de reflux (reflux:distillat) est compris entre 2:1 et 30:1.

**7.** Procédé selon une ou plusieurs des revendications 1 à 5, caractérisé en ce que le rapport reflux:distillat est compris entre 4:1 et 25:1.

**8.** Procédé selon une ou plusieurs des revendications 1 à 7, caractérisé en ce que le rapport d'alimentation de l'agent d'extraction au mélange de m- et p-dichlorobenzène est compris entre 4:1 et 40:1.

**9.** Procédé selon une ou plusieurs des revendications 1 à 7, caractérisé en ce que le rapport d'alimentation de l'agent d'extraction au mélange des dichlorobenzènes est compris entre 9:1 et 30:1.

**10.** Procédé selon une ou plusieurs des revendications 1 à 9, caractérisé en ce que l'on associe la rectification avec extraction à une cristallisation par fusion.